# EUROPEAN PATENT SPECIFICATION

(11) **EP 1 981 433 B1**
(45) Date of publication and mention of the grant of the patent: **10.08.2011**
(21) Application number: 06850350.7
(22) Date of filing: 21.12.2006
(51) Int. Cl.: A61F 2/90

(54) **FLEXIBLE EXPANDABLE STENT**
FLEXIBLER DEHNBARER STENT
STENT EXTENSIBLE FLEXIBLE

(30) Priority: 25.01.2006 US 252669; 25.01.2006 US 252668; 28.08.2006 US 823692 P; 13.09.2006 US 825434 P; 20.12.2006 US 613443
(43) Date of publication of application: 22.10.2008
(73) Proprietor: Cornova, Inc., Burlington, MA 01803 (US)
(72) Inventor: FLIEDNER, Thilo U., 81541 München (DE); SAHAGIAN, Richard, Burlington, MA 01803 (US); RYAN, S. Eric, Hopkinton, MA 01748 (US)
(74) Representative: Greenwood, John David
(86) International application number: PCT/US2006/062478
(87) International publication number: WO 2007/102926

(56) References cited:
- WO-A-00/03661
- WO-A-2006/005026
- US-A- 5 669 924
- US-A1- 2002 042 648
- US-B1- 6 193 747
- US-B1- 6 398 805

## Description

### BACKGROUND OF THE INVENTION

### 1. Field of the Invention

Embodiments of the present invention relate to medical stents which are implantable devices for propping open and maintaining the patency of vessels and ducts in the vasculature of a human being.

### 2. Description of the Related Art

Stents are implantable prosthesis used to maintain and/or reinforce vascular and endoluminal ducts in order to treat and/or prevent a variety of medical conditions. Typical uses include maintaining and supporting coronary arteries after they are opened and unclogged, such as through an angioplasty operation. A stent is typically deployed in an unexpanded or crimped state using a catheter and, after being properly positioned within a vessel, is then expanded into its final shape (such as with an expandable balloon incorporated into the catheter).

As a foreign object inserted into a vessel, a stent can potentially impede the flow of blood. This effect can also be exacerbated by the undesired growth of tissue and on and around the stent, potentially leading to complications including thrombosis and restenosis. Thus, stents are manufactured to minimize impedance of a vessel while being capable of maintaining their expanded state. Typical stents have the basic form of an open-ended tubular element supported by a mesh of thin struts with openings formed therein between. Designs typically include strong, flexible, and malleable base materials and, in order to resist excessive tissue growth, often include bio-compatible surface materials such as inert metals and/or various polymers. Other stent technologies include incorporating anti-growth drugs into the stent surface for timed-release elution.

In addition to being strong and resistant to undesired growth factors, most stents are manufactured to be reliably deformable in crimped and deployed states. Prior to deployment, a stent is generally crimped and secured about an expandable balloon at the distal end of a catheter. When inserted into position and expanded, the stent should preferably form a smoothly defined tubular structure aligned with the vessel walls. However, many commercially available stents suffer from problems including uneven expansion, failure to retain shape after expansion, corrosion, flaking, cracking, and other strut and surface imperfections.

Irregular expansion of stents is often caused by strut designs in which the radial force required to expand the stent is inconsistent across its length and/or about its circumference. This can occur in some designs at the endpoints because of a sudden decrease in longitudinal support at these terminating points, resulting in a "dog boning" effect during expansion. Many stents, because of too much lateral support, also do not bend and adapt well to curvatures in vessels. Another common problem of stent designs is where the stent shortens (or foreshortens) during radial expansion, producing an abrasive force against a vessel's walls.

Still other stents, because of surface coatings that are not well bonded or stable, tend to flake or crack during expansion or after exposure to internal body fluids. The effects of flaking or cracking of surface materials, which create a less smooth surface and can also substantially negate anti-growth properties, may even cause a serious blockage resulting in death. Many drug-eluding stents presently have drug-embedded polymer surfaces with these problems.

US 2002042648 describes a stent, in particular a coronary stent, for expansion from a first condition into an expanded second condition in which it holds a vessel in an expanded state, comprising a tubular body whose peripheral surface is formed by a number of annular support portions comprising bar elements which are connected in the longitudinal direction of the stent by way of connecting bars, wherein the bar elements (3) of at least a first support portion extend in a meander configuration in the peripheral direction of the stent and the bar element portions adjoining a turning point are arranged in a V-shape in the first condition of the stent, and wherein the bar element portions of the first support portion extend curvedly in a first direction in the longitudinal direction of the stent.

US 6193747 describes a stent which has a tubular flexible body with the wall having a web structure. The web structure of the wall has cells formed by a pair of adjacent sinuous web patterns that are interconnected by linking elements to form elastic elements.

WO 2006005026 describes expandable intraluminal medical devices for use within a body passageway or duct, and more particularly to an optimized stent having asymmetrical strut and loop members, wherein at least one pair adjacent radial strut members have unequal axial lengths.

WO 0003661 describes a stent having a unitary structure and having a principal axis and a succession of axially aligned tubular stages, at least some of those stages being defined by a regular pattern having consecutive loops closed on themselves, with a succession of hairpin-shaped apices which are connected consecutively, in pairs, by a branch, each branch having two inflexion points defining a double inflexion region, the loops being arranged in phase from one stage to an adjacent stage and being connected to one another by connecting bars linking the apex region of a loop of a given stage to the region of the inflexion points of a loop corresponding to it axially, in a preceding stage or in a following stage, in an alternating manner on the perimeter of said given stage.

US 6398805 describes a vascular or endoluminal stent which has low surface friction for ease of navigating a vessel, duct or tract of a patient and having the features of the preamble of claim 1. The stent is configured as a tubular element of biocompatible material having a longitudinal axis, open ends and a multiplicity of openings of generally common shape and size through its wall throughout its length. The openings are bounded by a network of tangentially interconnected, continuous, predominantly longitudinally oriented curvilinear struts, without discontinuity, forming a sidewall of the tubular element.

The present invention is characterized by the features of the characterizing portion of claim 1.

The present invention is as claimed in the claims.

Embodiments of the present invention relate to medical stent assemblies comprised of elongated tubular patterns of metal capable of expanding and propping open a vessel or duct within a living, human being. An aspect of the present invention comprises a plurality of circumferential arrays of switchback loops; as in the manner of an "arcuately shaped hairpin-like" curve or bend. The plurality of those circumferential arrays of switchback loops or hairpin-like curves are spaced apart from one another along the longitudinal axis of the stent.

Each adjacent circumferential array of loops is joined to its longitudinally adjacent circumferential array of loops by two or more arcuate cross-links. The cross-links generally smoothly extend the arcuate curvature of the tip portion. Each of those cross-links extends from a mid-portion of a curved section of arch of a switchbacks loop to the tip portion of the curved bend on a generally longitudinally adjacent curved switchback loop.

In an aspect of the present Invention, adjacent cicumferential arrays of switchback loops and cross-links form expansive circumferenially disposed "open cell" spaces that generally appear in a: "palm tree"-shaped form as viewed from a flattened radially inward directed perspective. The expansive "open cell" spaces, as described hereinbelow, easily permit a second stent assembly to be passed therethrough and expanded outwardly as in dual branched in a vessel bifurcation.

In embodiments of the invention, the strut width can be, for example, between about 50 to 80 microns, 80 to 100 microns, or 110 to 150 microns depending on, for example, the target vessel size (i.e., small, medium, or large).

After the insertion of an embodiment of the present invention, and during expansion of the adjacent circumferential loops of each array, the two or more cross-links between adjacent circumferential arrays can pivot, as viewed radially inwardly, so as to rotate from an oblique orientation with respect to alignment with longitudenial axis of the stent assembly, to an orientation which is "more parallel" to that longitudinal axis of that stent assembly. Such expansion and movement of the circumferential loops and pivoting of the cross-links can help forestall shortening of the length of the stent assembly as it expands within the vasculature of a patient.

A minimal number (e.g. two) of cross-links between longitudinally adjacent circumferential arrays of loops can add to the flexibility and adaptability of that stent assembly in the curved vasculature of a patient. Similarly, the un-tethered adjacent bends in the respective circumferential arrays can allow for substantially uniform strength over the length of the stent assembly, permitting substantially uniform expansion while avoiding such effects as "dog boning" or foreshortening of that stent assembly during expansion within a vessel.

In embodiments of the invention, the pattern of corresponding, generally longitudinally aligned hairpin-like curves can also minimize or prevent the likelihood of detrimental interfering contact between non-contiguous aspects of the switchback loops when in various expanded and unexpanded states.

In an embodiment of the present invention, the annular arrays are comprised of a cobalt-chromium alloy. The cobalt-chromium base can be layered with inert biocompatible materials, including gold, silver, platinum, or various non-metallic polymers. Surface layers may further be comprised of biologically active materials, including anti growth drugs for timed release elution.

In a further embodiment of the present invention, a relatively thin, substantially uniform biocompatible metallic layer is ion-implanted onto a cobalt-chromium base such as, for example, with the use of a magnetron having unbalanced magnetic fields.

Each of the circumferential array of webs may be comprised of a pattern of first lengthwise sized bends and a second pattern of lengthwise-elongated sized bends. The lengthwise-elongated sized bends are preferably longitudinally longer than the first lengthwise sized bends.

In an embodiment of the invention, the longitudinally adjacent arrays of generally arcuately shaped hairpin-like curved bends or webs are connected to one another by a cross-link arrangement connected to diagonally adjacent bends of the longitudinally adjacent arrays of bends or webs. In an embodiment of the invention, the cross-link an-angement between longitudinally adjacent arrays preferably consists of two cross-links connecting adjacent arrays. The cross-links can be connected between the lengthwise-elongated sized bends of the adjacent arrays. The lengthwise-elongated sized bends connected by a cross-link can be spaced diagonally adjacent one another in longitudinally adjacent arrays of bends. A circumferential gap is arranged between circumferentially adjacent cross-links, to permit proper bending of the stent assembly in the vasculature of a being. In an embodiment of the invention, the circumferential gap is of "Palm Tree" shape in a radially inwardly directed view, to promote flexibility to the stent assembly. The cross-links between adjacent circumferential arrays can be pivotable into general longitudinal alignment with the longitudinal axis of the stent assembly during expansion of the stent assembly in a body lumen. The assembly may have a substrate surface comprised of, for example, cobalt-chromium. The assembly may have a bio-compatible surface layer thereon. The bio-compatible surface layer can comprise, for example, platinum. The surface layer can comprise graduated sublayers of platinum and palladium atoms. The sublayers can include an adhesion layer comprised substantially of palladium, a transition layer in which the ratio of palladium content is gradually decreased and the ratio of platinum content is gradually increased, with an outermost layer comprised substantially of platinum. The adhesion and the palladium layer and the outermost layer can have a thickness for example, of between about 50nm (500 angstroms) and about 500nm (5000 angstroms). The at least one of the adhesion layers and the outermost layer have a thickness for example of no greater than about 250nm (2500 angstroms). The platinum layer and the palladium layer can be implanted onto the stent assembly preferably by, for example, the method of: ion-bombardment.

In an aspect of the invention, a stent assembly for implantation into a human vessel, is comprised of an elongated collection of circumferentially extending curved webs, each of the circumferentially extending curved webs being in generally corresponding alignment with one another. Each of the circumferentially extending curved webs may be adjacently connected by a pair of cross-links. Each of the pairs of cross-links can be spaced diagonally across from one another, between adjacent circumferentially extending curved webs, Each pair of cross-links preferably defines a "Palm Tree" shaped arcuate gap, as viewed from a flattened radial perspective, between adjacent circumferentially extending curved webs. Each of the circumferentially extending curved webs consists of at least two sets of curves each of which is comprised of a pair of a longitudinally short first bend members separated by a single longitudinally elongated second bend member at every third bend position on an array. The cross-links connecting the longitudinally adjacent circumferentially extending curved webs may be attached between diagonally adjacent second bend members on longitudinally adjacent circumferentially curved webs. The cross-links can be pivotable between longitudinally adjacent circumferentially curved webs during expansion of the stent assembly.

An embodiment of the invention may be used in a method (not according to the present invention comprising one or more of the following steps including, placing a first stent assembly into the body vessel at its vessel bifurcation, and into a first arm of the vessel bifurcation; placing a second stent assembly into the first stent assembly in the vessel; and directing the second stent assembly at least partway through an opening in a wall portion of the first stent assembly and into a second arm of the vessel bifurcation, the first and second stent assemblies each having a longitudinal axis. The opening in the wall portion of the first stent assembly may comprise a circumferentially directed gap between longitudinally adjacent curved hairpin-like curves. The circumferentially directed gap may be comprised of a generally "Palm Tree" shaped open cell. The method may include placing an obliquely disposed cross-link across the circumferentially directed gap between longitudinally adjacent curved hairpin-like curves, and pivoting the obliquely disposed cross-link into a generally parallel alignment with the longitudinal axis of the stent assembly.

The method of stenting a bifurcated vessel of a patient may comprise one or more of the following steps comprising: placing a first stent assembly into the vessel at its vessel bifurcation, and into a first arm of the vessel bifurcation; placing a second stent assembly into the first stent assembly in the vessel; and directing the second stent assembly at least partway through an opening in a wall portion of the first stent assembly and into a second arm of the vessel bifurcation, the first and second stent assemblies each having a longitudinal axis. The opening in the wall portion of the first stent assembly preferably comprises a circumferentially directed gap between longitudinally adjacent curved hairpin-like curves. The circumferentially directed gap preferably comprises a generally "Palm Tree" shaped open cell. The method may include placing an obliquely disposed cross-link across the circumferentially directed gap between longitudinally adjacent curved hairpin-like curves, pivoting the obliquely disposed cross-link into a generally parallel alignment with the longitudinal axis of the stent assembly, leaving at least a portion of the second stent assembly within and in fluid communication with the first stent assembly at the bifurcation, aligning a circumferential gap of the first stent assembly with a circumferential gap of the second stent assembly to permit the first and second stent assemblies interdigitation, aligning a circumferential gap of the second stent assembly with a central lumen of the first stent assembly to facilitate fluid communication therebetween, inserting the first stent assembly and the second stent assembly into a body vessel, simultaneously, overlapping a longitudinal portion of the second stent assembly with a longitudinal portion of the first stent assembly during their simultaneous introduction into a body vessel, directing a distal portion of said second stent assembly through a circumferential gap in the first stent assembly subsequent to the first stent assembly and the second stent assembly being simultaneously introduced into a body vessel.

An embodiment of the invention also comprises a flexible, expandable stent assembly for introduction into and stenting of a bifurcation in a body vessel, comprising: a first generally cylindrically shaped channel, having a longitudinal axis, and having a plurality of openings therein, the openings being defined by longitudinally aligned circumferential arrays of arcuately shaped, generally hairpin-like curved webs or bends of metal; and a second generally cylindrically shaped channel, having a longitudinal axis, and having a plurality of openings therein, the openings being defined by longitudinally aligned circumferential arrays of arcuately shaped, generally hairpin-like curved webs or bends of metal, the second generally cylindrically shaped channel extending at least partially longitudinally within the first generally cylindrically shaped channel.

An embodiment of the invention may be used in a method (not according to the present invention comprising one or more of the following steps of: expanding radially outwardly a plurality of longitudinally connected annular arrays of hairpin-like shaped webs of first and second length bends of metal, connecting the annular arrays of hairpin-like shaped webs by at least two cross-links spaced between a pair of second length bends arranged in neighboring arrays of the annular arrays of webs; and re-orienting the first and second length bends as the stent assembly expands within a body lumen to maintain original body length of the stent assembly; :re-orienting a cross-link disposed between the neighboring second length bends. The first and second bends and the cross-links can be correspondingly smoothly curved.

### Brief Description of the Drawings

The objects and advantages of the present invention will become more apparent when viewed in conjunction with the following drawings, in which:

Figure 1 is a longitudinal presentation, in a flat or "planar" array, of a stent assembly in an embodiment of the present invention;

Figure 1A is an enlarged view, in plan, of a portion of a circumferential array of arcuately shaped hairpin-like bends of the stent assembly shown in Fig. 1;

Figure 2A is a side elevational view of a stent assembly in an embodiment of the present invention in a cylindrical configuration;

Figure 2B is a perspective view of the stent assembly of Fig. 2A, again in a cylindrical configuration; and

Figure 3 is a perspective view of two stent assemblies in accordance with an embodiment of the present invention shown interdigitated in a vessel bifurcation.

### Detailed Description of Embodiments of the Invention

The accompanying drawings are described below, in which example embodiments in accordance with the present invention are shown. Specific structural and functional details disclosed herein are merely representative. This invention may be embodied in many alternate forms and should not be construed as limited to example embodiments set forth herein.

Accordingly, specific embodiments are shown by way of example in the drawings. Like numbers refer to like elements throughout the description of the figures.

It will be understood that, although the terms first, second, etc. may be used herein to describe various elements, these elements should not be limited by these terms. These terms are used to distinguish one element from another. For example, a first element could be termed a second element, and, similarly, a second element could be termed a first element, without departing from the scope of the present disclosure. As used herein, the term "and/or" includes any and all combinations of one or more of the associated listed items.

It will be understood that "adjacent" does not necessarily imply contact but may connote an absence of the same kind of element(s) therein between "adjacent" elements.

It will be understood that when an element is referred to as being "on," "connected to" or "coupled to" another element, it can be directly on, connected to or coupled to the other element or intervening elements may be present. In contrast, when an element is referred to as being "directly on," "directly connected to" or "directly coupled to" another element, there are no intervening elements present. Other words used to describe the relationship between elements should be interpreted in a like fashion (e.g., "between" versus "directly between," "adjacent" versus "directly adjacent," etc.).

The terminology used herein is for the purpose of describing particular embodiments and is not intended to be limiting of the invention. As used herein, the singular forms "a," "an" and "the" are intended to include the plural forms as well, unless the context clearly indicates otherwise. It will be further understood that the terms "comprise," "comprises," "comprising," "include," "includes" and/or "including," when used herein, specify the presence of stated features, integers, steps, operations, elements, and/or components, but do not preclude the presence or addition of one or more other features, integers, steps, operations, elements, components, and/or groups thereof.

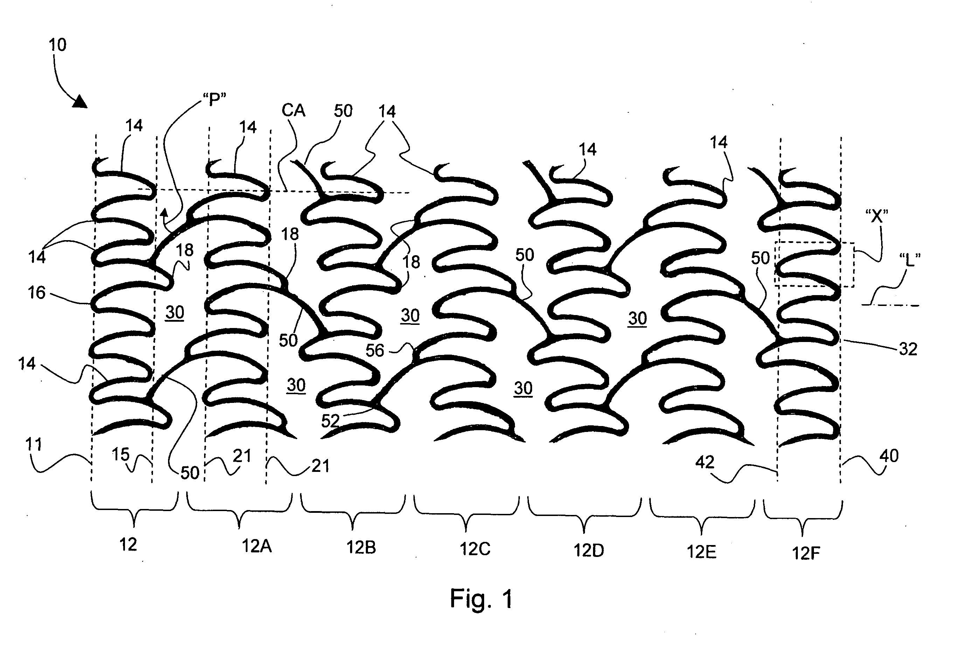

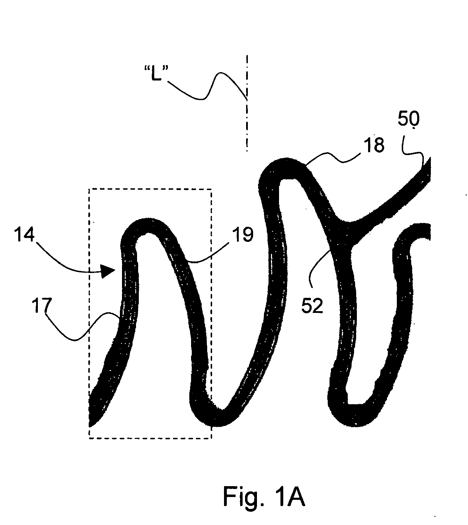

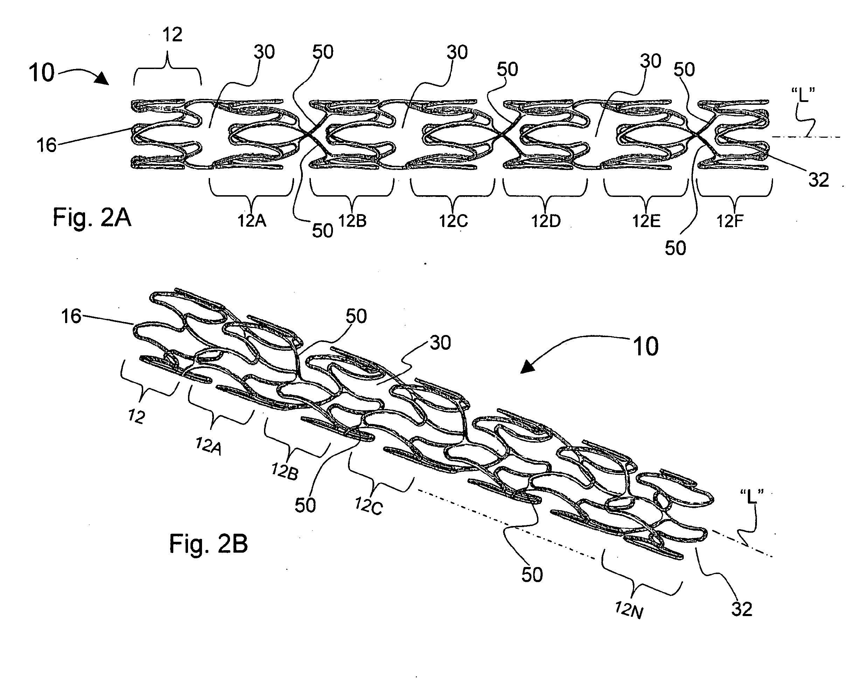

Referring now to the drawings in detail, and particularly to figure 1, a stent assembly 10 in accordance with an embodiment of the present invention is represented in a flat or planar configuration for case of understanding. The medical stent assembly 10 is comprised of an elongated tubular pattern of metal capable of expanding and propping open a vessel or duct within a living being, as represented in its cylindrical form, in figures 2A and 2B. The stent assembly 10 comprises a plurality of web-like, circumferential arrays 12, 12A etc. of switchback bends or loops 14, generally in the manner of an arcuately shaped "hairpin-like" curve, as indicated within the dashed rectangle "X" shown in figure 1. A loop or curve 14 is shown in an enlarged representation in figure 1A. The arcuately shaped "hairpin-like" curves 14 have a smoothly curved concave side 17 and a smoothly curved convex side 19. Thus such concave and convex sides 17 and 19 are correspondingly curved circumferentially, that is, curved in the "same direction" along the circumference of the stent, in their definition of each individual loop or curve 14.

There are, for example, a plurality of those circumferential arrays of switchback loops or hairpin-like curves 12, 12A, 12B, 12C, 12D, 12E and 12F spaced apart from one another along the longitudinal axis "L" of the stent assembly 10, as shown in figures 1, 2A and 2B. The loops or bends 14 at a first end 16 of the stent assembly 10 in the first circumferential array 12 thereat, are all generally in peripheral alignment with one another, as indicated by their edge in alignment with the dashed line "11". Elongated loops 18 on the inwardly directed side of the first or leftmost circumferential array 12 of every third of the switchbacks or hairpin-like curves or loops 14 extend longitudinally beyond of the peripheral border alignment, as indicated by the dashed line "15" of their adjacent bends. Further, the elongated loops 18 in each of the circumferential arrays 12, 12A etc comprising at least every third of the switchbacks or hairpin-like curves or loops 14 may extend longitudinally beyond one or more of their peripheral border alignments, as indicated by the dashed lines "11 and 15" of their adjacent bends, in an exemplary manner, for the two leftmost arrays 12 and 12A.

A plurality of smoothly curved, arcuate cross-links 50 are arranged so as to connect diagonally adjacent elongated loops 18 between longitudinally adjacent arrays 12, 12A etc., of bends or curves 14. Those elongated loops 18 preferably comprise every third loop 14 as most easily seen in figure 1.

The second and successive circumferential arrays 12A, 12B etc, of switchback or hairpin-like curves or loops 14 are in generally corresponding longitudinal alignment with the switchback or hairpin-like curves or loops 14 of the first circumferential array 12 of loops 14 at the first end 16 of the stent assembly 10, as indicated by line CA, shown in figure 1 passing through the tips of the loops 14, which may be called "fronds" in keeping with a "Palm Tree" shape further described herein below. That is, a switchback or loop 14 of an Nth circumferential array 12N is in generally longitudinal alignment with a corresponding switchback or loop 14 in the N + I circumferential array 12N+1 of switchback or hairpin-like curves or loops 14.

Each adjacent circumferential array 12, 12A etc of loops or arcuately shaped hairpin-like curves 14 is joined to its longitudinally adjacent circumferential array 12A, 12B etc...of loops or hairpin-like curves 14 by at least two smoothly curved arcuate cross-links 50. Each of those cross-links 50 extends from a mid-portion 52 of a curved section of arch of an elongated switchback loop 18 to a tip portion 56 of the curved hairpin-like curve or bend 14 on a generally diagonally adjacent elongated curved switchback loop 18, as best represented in figure 1, and which may be seen in figures 2A and 2B. Those cross-links 50 extending from tip portions 56 are on the same longitudinal end of a circumferential array 12, 12A etc and those cross-links that extend from a mid-portion 52 are on the opposite longitudinal end of the circumferential array, which can help promote uniform expansion of the stent.

For various embodiments of the invention, the general pattern can be adapted for differently sized stents or stents of different strengths varied according to need. For example, the frequency or number of circumferential arrays may be varied and the number of hairpin-like curves or loops may be varied as necessary for each circumferential array. For example, embodiments of the pattern with six hairpin-like loops for each circumferential array can provide for a stent length of about 9 mm with three columns of circumferential arrays, a length of about 12 mm with four columns of circumferential arrays, a length of about 15 mm with five columns of circumferential arrays, etc... These embodiments can have, for example, initial outer diameters of about 2 mm, crimped inner diameters of about 0.7 mm, and deployed outer diameters of about 2.75 mm, 3.0 mm, 3.5 mm, or 4.0 mm.

The elongated switchback loops 18 in every series of peripherally adjacent bends on adjacent circumferential arrays 12 A etc. extend longitudinally beyond the bends or tips of their circumferentially adjacent hairpin-like curves 14, as indicated by the dashed lines 15, 21, and 42, shown in figure 1.

A generally semi-circumferentially extending annular, circumferentially elongated gap or space 30 between array 12 and longitudinally adjacent array 12A defined by their respective circumferential loops 14 and the arcuate cross-links 50 resembles the aforementioned branched "Palm Tree" configuration, most conspicuously shown in figure 1.

The last circumferential array 12F of switchback loops or hairpin-like curves 14 on the second end 32 of the stent assembly 10 has an edge array of bends 14 thereon which are generally in peripheral alignment with one another, as indicated by their common alignment with dashed line 40, as shown in figure 1. That last circumferential array 12F at the second end 32 of the stent assembly 10 also has elongate bends or elongate switchback loops 18, extending longitudinally beyond the peripheral edge of the adjacent switchback loops or hairpin-like curves 14 on that particular circumferential , array 12F, as indicated by their extending longitudinally "inwardly" beyond the dashed line 42, also shown in figure 1.

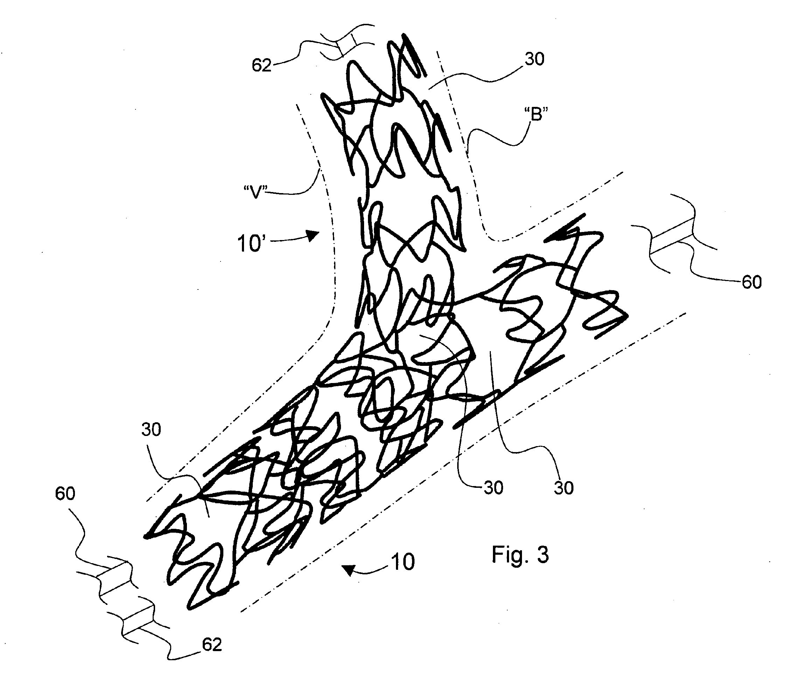

Thus, there are annular gaps 30 between adjacent circumferential arrays 12, 12A etc. of switchback loops or hairpin-like curves 14 comprising about 180 degrees of the peripheral space of the stent assembly 10 at that particular longitudinal location between adjacent arrays 12, 12A etc.. That 180 degree clear, open, circumferentially disposed, "Palm Tree" shaped "open cell" space 30 between adjacent circumferential arrays 12, 12A etc. generally comprises a "half periphery" of the stent assembly 10 which, as described hereinbelow in figure 3, permits a second stent assembly 10' to be passed therethrough and expanded outwardly as in a vessel bifurcation, because of the multiple longitudinally-dispersed, half-circumference "open cell" structure of each particular stent assembly 10 allowing such multiple stent assembly interdigitation. Further embodiments within the scope of this invention may include more than two annular "open cell" spaces or gaps between circumferential arrays 12, 12A etc of loops 14, depending upon the number of cross-links 50 dividing up each annular space between adjacent arrays 12, 12A etc. For example, one embodiment may extend the general pattern of open spaces 30 to comprise three annular "open spaces" or gaps 30, each one of which spans about a third of the periphery (about 120 degrees) of the stent assembly 10. In a further embodiment, a varying number (e.g. 2, 3 or more) of cross-links may be disposed between adjacent arrays 12, 12A etc. is contemplated, to provide any particular desired variation in bending and/or in receptability to through-wall penetration by several stent assemblies 10, 10' etc.

Referring now to Figure 3, the interdigitation of a first stent assembly 10' through a second stent assembly 10 within a body vessel bifurcation "B" is shown. Such a multiple stenting is made easier by virtue of the expansive circumferential "Palm Tree" shaped open cell spaces 30, as represented in figure 1. A minimized number of cross-links 50 (e.g. 2) between adjacent arrays 12, 12A etc of hairpin-like curves 14 promotes the curvature of each stent 10 and 10' for accommodating one another and for being penetrated by another stent 10' without significant interference, which is is highly beneficial to a patient needing such a bifurcation procedure. This double stenting at a bifurcated vessel "B" may be done one stent at a time, with the second stent 10' being directed though the longitudinal opening of the first stent assembly 10 then angularly directed through such a "Palm Tree" shaped side opening 30 which is in alignment with the further vessel "V", at the bifurcation "B" being stented.

The first and second stent assemblies 10 and 10' may be introduced on a common catheter (not shown for clarity), simultaneously. That is, each stent assembly 10 and 10' have an overlapping longitudinal relationship during their introduction into a body vessel and towards a bifurcation in that vessel. Upon reaching the bifurcation "B", the second stent assembly 10' may be directed outwardly, via a delivery wire 62 through a "Palm Tree" circumferential gap 30 in the first stent assembly 10. It is contemplated in one embodiment, that each stent assembly 10 and 10' may each have independently manipulable, co-linear delivery wires 60 and 62 to effectuate such steered manipulation of the second stent assembly 10' sideways and out of a circumferential "Palm Tree" gap 30 the first stent assembly 10, as represented in figure 3. In a further aspect of the present invention the second stent 10' may by of smaller diameter to facilitate relative movement therebetween. Further, the second stent assembly 10'in a bifurcation procedure of the present invention may be of shorter length to facilitate the stenting of a bifurcation B, or to accommodate only a relatively short branch needing stenting extending off of the parent vessel, to minimize any unnecessary overlap between the first and second stent assemblies 10 and 10'.

After the insertion of such a stent assembly 10 of the present invention in a vessel, bifurcated or otherwise, and upon expansion of the adjacent circumferential loops 14 of each array 12, 12A etc..., each of the cross-links 50 between adjacent circumferential arrays 12, 12A, etc... may in one embodiment, move to re-orient slightly or pivot, as viewed radially inwardly for example, and indicated by the arrow "P", in figure 1, so as to rotate or pivot from an oblique orientation with respect to its alignment with longitudinal axis "L" of the stent assembly 10, to an orientation which is "more parallel" to that longitudinal axis "L" of that stent assembly 10. Such a movement of those cross-links 50, helps forestall any shortening of the length of the stent assembly 10 as it expands within the vasculature of a patient. Such annular or circumferential disposition of the semi-circumferential gaps or spacings 30 during expansion of the stent assembly 10, and the rotation of the cross-links 50 however, remain in general circumferential disposed alignment with respect to the longitudinal axis of the stent assembly 10, and not obliquely angled with respect thereto. Such stent assembly 10 foreshortening during expansion thereof is however, primarily prevented by the expansive common circumferential and longitudinally directed deformation of the curves or bends 14 due to their unique curvilinear configuration, which comprises the structure being moved radially outwardly.

The minimal number of cross-links 50 between longitudinally adjacent circumferential arrays 12, 12A etc of loops 14 adds to the stent assembly's flexibility and adaptability of that stent assembly 10 in the curved vasculature of a patient. Similarly, the un-tethered adjacent bends 14 in the respective circumferential arrays 12, 12A etc. allows for substantially uniform radial strength over the length of the stent assembly 10 permitting substantially uniform expansion and avoidance of such effects as "dog boning" or the foreshortening of that stent assembly 10 within a patient.

In an aspect of the use of the stent assembly of the present invention, the extension of a first stent assembly with a second stent assembly is provided for by overlapping a portion of the longitudinal ends (e.g.. first end 16 or second end 32 as shown in figure 1) of stent assemblies in accordance with the strut design of the present invention, to create an arrangement known as "kissing stents." A first stent assembly 10 is inserted and expanded into a vessel. A second stent assembly 10 is then inserted through the longitudinal opening of the first stent assembly so that it partially overlaps a longitudinal section of the first stent assembly 10, after which the second assembly is expanded in place. The second stent assembly can be of a smaller initial diameter to better accommodate fitting within the first stent assembly 10 and/or for simultaneous deployment/expansion (wherein the stents are initially overlapping and are inserted together). A minimal amount of strut structure embodied in each stent assembly of the present invention reduces the likelihood of interaction with tissue material along the overlapping portions of their outer circumferences.

In an embodiment of the present invention, the struts of annular arrays 12, 12A etc. and cross-links 50 are comprised of a cobalt-chromium. The struts may be layered with inert biocompatible materials, including gold, silver, platinum, or various non-metallic polymers. Surface layers may further be comprised of biologically active materials, including anti growth drugs for timed release elution, such as those described in US Patent # 6,120,536 by Ding et al.

The thicknesses of the struts can be optimized to promote flexibility, minimal surface contact, and the expansiveness of the spaces between struts. In an embodiment of the invention, the struts are of a thickness of between about 60 and 100 microns and, at non-connecting joints, can average about 80 microns in width which can, for example, be suitable for medium sized vessels (from 3mm to less than 4 mm in diameter). In another embodiment of the invention, the struts are of a thickness of between about 50 and 80 microns and, at non-connecting joints, average about 65 microns in width which can, for example, be suitable for smaller sized vessels (less than 3 mm in diameter). In another embodiment of the invention, the strut are of a thickness of between about 110 and 150 microns and, at non-connecting joints, can average about 130 microns in width which can, for example, be suitable for larger sized vessels (4 mm in diameters and larger).

In an embodiment of the present invention, a biocompatible metallic layer is ion-implanted onto a cobalt-chromium base, such as, for example, through methods which use a magnetron having unbalanced magnetic fields as described in pending US Patent Application # 09/999,349 by Sahagian, published Sept. 26, 2006 as US Patent Application # 2002/0138130A1) and pending US Patent Application 60/823,692 by Sahagian, et al.

In a further embodiment of the present invention, gradations of platinum and palladium ions are implanted onto a cobalt chromium base through variations of these methods to produce an adhesion layer of substantially palladium, a transition layer of increasing platinum content and decreasing palladium content and a bio-compatable capping layer of substantially platinum. In further embodiments of the present invention, the palladium and platinum layers can be from about 100 angstroms and up to about 5,000 angstroms thick, preferably greater than for example, about 500 angstroms thick, and less than about 2,500 angstroms, such that they are optimized to maximize the smoothness and stability of the layers. The thicknesses may depend upon various parameters, including the size and projected expansion of the stent assembly.

## Claims

1. A flexible, expandable, elongated stent assembly (10) comprising:
a generally cylindrically shaped channel, having a longitudinal axis (L), and having a plurality of openings (30) therein, said openings (30) being defined by a structure of longitudinally aligned circumferential switch-back arrays (12, 12A, ....) of arcuately shaped and smoothly curved hairpin-like bends (14) and in which adjacent arrays are connected by arcuate cross-links (50); and wherein:
each of said cross-links (50) extends from a mid-portion of a longitudinally extending curved section of a bend to the tip portion of a bend of a longitudinally adjacent array; and
the connection of each cross-link (50) to said tip portion of a bend of a longitudinally adjacent array generally smoothly extends the arcuate curvature of said bend of the longitudinally adjacent array (12, 12A, ....).

2. The flexible, expandable stent assembly (10) as recited in claim 1, wherein the cross-links (50) are connected to diagonally disposed bends (14) of said longitudinally adjacent arrays of bends (14) or webs.

3. The flexible, expandable stent assembly (10) as recited in claim 2, wherein said diagonally disposed bends (14) are diagonally adjacent.

4. The flexible, expandable stent assembly (10) as recited in claim 3, wherein said cross-link arrangement between longitudinally adjacent arrays (12, 12A, ....) comprises two or more cross-links (50) connecting each adjacent array.

5. The flexible, expandable stent assembly (10) as recited in claim 4, wherein said two or more cross-links (50) connecting each adjacent arrays consists of two cross-links (50).

6. The flexible, expandable stent assembly (10) as recited in claim 1, wherein a substantial portion of each of said curved bends (14) form arcs of generally the same orientation with respect to the circumference of said stent assembly.

7. The flexible, expandable stent assembly (10) as recited in claim 1, wherein said assembly comprises a substrate with a thickness of between about 50 and 80 microns.

8. The flexible, expandable stent assembly (10) as recited in claim 1, wherein said assembly has a substantially biocompatible surface layer thereon.

9. The flexible, expandable stent assembly (10) as recited in claim 8, wherein said bio-compatible surface layer comprises a substantially biocompatible metal.

10. The flexible, expandable stent assembly (10) as recited in claim 9, wherein said substantially biocompatible metal is selected from the group consisting of platinum, gold and silver.

11. The flexible, expandable stent assembly (10) as recited in claim 8, wherein said biocompatible surface layer comprises graduated sub-layers incorporating metals adhesive to the substrate surface of said stent.

12. The flexible, expandable stent assembly (10) as recited in claim 11, wherein said sub-layers include an adhesion layer comprised substantially of palladium, a transition layer in which the ratio of palladium content is gradually decreased and the ratio of platinum content is gradually increased, with an outermost layer comprised substantially of platinum.

13. The flexible, expandable stent assembly (10) as recited in claim 9, wherein said biocompatible surface layer has a thickness of between 10nm (100 angstroms) and about 500nm (5000 angstroms).

14. The flexible, expandable stent assembly (10) as recited in claim 13, wherein
said biocompatible surface layer has a thickness of no greater than 250nm (2500 angstroms).

15. The flexible, expandable stent assembly (10) as recited in claim 9, wherein said biocompatible surface layer is implanted onto said stent assembly (10) using the method of ion-bombardment.

16. A flexible, expandable stent assembly (10) for stenting of a bifurcation in a body vessel, comprising:
a first generally cylindrically shaped channel according to claim 1; and
a second generally cylindrically shaped channel according to claim 1,
said second generally cylindrically shaped channel extending at least partially longitudinally within said first generally cylindrically shaped channel.

## Patentansprüche

1. Flexible, erweiterbare, verlängerte Stentanordnung (10), welche Folgendes umfasst:
einen im Allgemeinen zylinderförmigen Kanal, welcher eine Längsachse (L) und mehrere Öffnungen (30) darin aufweist, wobei die Öffnungen (30) durch eine Struktur längs ausgerichteter umlaufender Rückschalt-Arrays (12, 12A, ...) bogenförmiger und leicht gekrümmter Haarnadelbiegungen (14) definiert sind und bei der angrenzende Arrays durch bogenförmige Querverbindungen (50) verbunden sind; und wobei
sich jede der Querverbindungen (50) von einem Mittelteil eines sich längs erstreckenden gekrümmten Abschnittes einer Biegung hin zum Spitzenteil einer Biegung eines längs angrenzenden Arrays erstreckt; und
der Anschluss jeder Querverbindung (50) an den Spitzenteil einer Biegung eines längs angrenzenden Arrays die bogenförmige Krümmung der Biegung des längs benachbarten Arrays (12, 12A, ...) im Allgemeinen leicht verlängert.

2. Flexible, erweiterbare Stentanordnung (10) nach Anspruch 1, wobei die Querverbindungen (50) mit diagonal angeordneten Biegungen (14) der längs angrenzenden Arrays von Biegungen (14) oder Stegen verbunden sind.

3. Flexible, erweiterbare Stentanordnung (10) nach Anspruch 2, wobei die diagonal angeordneten Biegungen (14) diagonal angrenzend sind.

4. Flexible, erweiterbare Stentanordnung (10) nach Anspruch 3, wobei die Querverbindungsanordnung zwischen längs angrenzenden Arrays (12, 12A, ...) zwei oder mehr Querverbindungen (50) umfasst, welche jedes angrenzende Array verbinden.

5. Flexible, erweiterbare Stentanordnung (10) nach Anspruch 4, wobei die zwei oder mehr Querverbindungen (50), welche jedes angrenzende Array verbinden, aus zwei Querverbindungen (50) bestehen.

6. Flexible, erweiterbare Stentanordnung (10) nach Anspruch 1, wobei ein wesentlicher Teil jeder der gekrümmten Biegungen (14) Bögen mit der im Allgemeinen gleichen Ausrichtung in Bezug auf den Umfang der Stentanordnung bildet.

7. Flexible, erweiterbare Stentanordnung (10) nach Anspruch 1, wobei die Anordnung ein Substrat mit einer Dicke zwischen etwa 50 und 80 Mikronen umfasst.

8. Flexible, erweiterbare Stentanordnung (10) nach Anspruch 1, wobei die Anordnung eine im Wesentlichen biokompatible Oberflächenschicht darauf aufweist.

9. Flexible, erweiterbare Stentanordnung (10) nach Anspruch 8, wobei die biokompatible Oberflächenschicht ein im Wesentlichen biokompatibles Metall umfasst.

10. Flexible, erweiterbare Stentanordnung (10) nach Anspruch 9, wobei das im Wesentlichen biokompatible Metall aus der Gruppe bestehend aus Platin, Gold und Silber ausgewählt ist.

11. Flexible, erweiterbare Stentanordnung (10) nach Anspruch 8, wobei die biokompatible Oberflächenschicht abgestufte Unterschichten umfasst, welche Metalle enthalten, die an der Substratfläche des Stents haften.

12. Flexible, erweiterbare Stentanordnung (10) nach Anspruch 11, wobei die Unterschichten eine Haftschicht beinhalten, welche im Wesentlichen aus Palladium besteht, eine übergangsschicht, in welcher das Verhältnis des Palladiumgehaltes allmählich abnimmt und das Verhältnis des Platingehaltes allmählich zunimmt, 1 sowie eine äußerste Schicht, die im Wesentlichen aus Platin besteht.

13. Flexible, erweiterbare Stentanordnung (10) nach Anspruch 9, wobei die biokompatible Oberflächenschicht eine Dicke zwischen 10 nm (100 Ångström) und etwa 500 nm (5000 Ångström) aufweist.

14. Flexible, erweiterbare Stentanordnung (10) nach Anspruch 13, wobei die biokompatible Oberflächenschicht eine Dicke von nicht mehr als 250 nm (2500 Ångström) aufweist.

15. Flexible, erweiterbare Stentanordnung (10) nach Anspruch 9, wobei die biokompatible Oberflächenschicht mit Hilfe des Ionenbeschussverfahrens auf die Stentanordnung (10) implantiert wird.

16. Flexible, erweiterbare Stentanordnung (10) für die Stent-Implantation in einer Gabelung in einem Körpergefäß, welche Folgendes umfasst:
einen ersten im Allgemeinen zylinderförmigen Kanal nach Anspruch 1; und
einen zweiten im Allgemeinen zylinderförmigen Kanal nach Anspruch 1,
wobei sich der zweite im Allgemeinen zylinderförmige Kanal zumindest teilweise längs innerhalb des ersten im Allgemeinen zylinderförmigen Kanals erstreckt.

## Revendications

1. Ensemble stent extensible flexible allongé (10), comprenant :
un conduit de forme généralement cylindrique, ayant un axe longitudinal (L) et comportant une pluralité d'ouvertures (30), lesquelles ouvertures (30) sont définies par une structure à réseaux en lacets circonférentiels alignés dans le sens longitudinal (12, 12A, ...), constitués de courbes en épingles à cheveux de forme arquée et doucement incurvées (14), dans laquelle des réseaux adjacents sont reliés par des attaches arquées (50) ; et dans lequel :
chacune desdites attaches (50) s'étend depuis une partie centrale d'un segment incurvé d'un lacet s'étendant dans le sens longitudinal jusqu'à la partie d'extrémité d'un lacet d'un réseau adjacent dans le sens longitudinal ; et
le raccordement de chaque attache (50) à ladite partie d'extrémité d'un lacet d'un réseau adjacent dans le sens longitudinal prolonge généralement en douceur la courbure arquée dudit lacet du réseau adjacent dans le sens longitudinal (12, 12A,...).

2. Ensemble stent extensible flexible (10) selon la revendication 1, dans lequel les attaches (50) sont reliées à des lacets disposés en diagonale (14) desdits réseaux ou nappes de lacets adjacents dans le sens longitudinal (14).

3. Ensemble stent extensible flexible (10) selon la revendication 2, dans lequel lesdits lacets disposés en diagonale (14) sont diagonalement adjacents.

4. Ensemble stent extensible flexible (10) selon la revendication 3, dans lequel ledit agencement d'attaches entre des réseaux adjacents dans le sens longitudinal (12, 12A, ...) comprend deux ou plusieurs attaches (50) reliant chaque réseau adjacent.

5. Ensemble stent extensible flexible (10) selon la revendication 4, dans lequel lesdites deux ou plusieurs attaches (50) reliant des réseaux adjacents comprennent deux attaches (50).

6. Ensemble stent extensible flexible (10) selon la revendication 1, dans lequel une partie substantielle de chacun desdits lacets incurvés (14) forme des arcs ayant généralement la même orientation par rapport à la circonférence dudit ensemble stent.

7. Ensemble stent extensible flexible (10) selon la revendication 1, dans lequel ledit ensemble comprend un substrat ayant une épaisseur dans la plage de 50 à 80 microns environ.

8. Ensemble stent extensible flexible (10) selon la revendication 1, dans lequel ledit ensemble comporte une couche superficielle essentiellement biocompatible.

9. Ensemble stent extensible flexible (10) selon la revendication 8, dans lequel ladite couche superficielle biocompatible comprend un métal essentiellement biocompatible.

10. Ensemble stent extensible flexible (10) selon la revendication 9, dans lequel ledit métal essentiellement biocompatible est choisi dans le groupe constitué par le platine, l'or et l'argent.

11. Ensemble stent extensible flexible (10) selon la revendication 8, dans lequel ladite couche superficielle biocompatible comprend des sous-couches graduées incorporant des métaux qui adhèrent à la surface du substrat dudit stent.

12. Ensemble stent extensible flexible (10) selon la revendication 11, dans lequel lesdites sous-couches comprennent une couche d'adhérence contenant essentiellement du palladium, une couche de transition dans laquelle le ratio de la teneur en palladium diminue graduellement et le ratio de la teneur en platine augmente graduellement, avec une couche la plus à l'extérieur contenant essentiellement du platine.

13. Ensemble stent extensible flexible (10) selon la revendication 9, dans lequel ladite couche superficielle biocompatible a une épaisseur dans la plage de 10 nm (100 angströms) à environ 500 nm (5000 angströms).

14. Ensemble stent extensible flexible (10) selon la revendication 13, dans lequel ladite couche superficielle biocompatible a une épaisseur non supérieure à 250 nm (2500 angströms).

15. Ensemble stent extensible flexible (10) selon la revendication 9, dans lequel ladite couche superficielle biocompatible est implantée sur ledit ensemble stent (10) en utilisant le procédé de bombardement ionique.

16. Ensemble stent extensible flexible (10) destiné à être implanté dans une bifurcation d'un vaisseau sanguin, comprenant :
un premier conduit de forme généralement cylindrique selon la revendication 1 ; et
un deuxième conduit de forme généralement cylindrique selon la revendication 1, lequel deuxième conduit de forme généralement cylindrique s'étend au moins en partie dans le sens longitudinal à l'intérieur dudit premier conduit de forme généralement cylindrique.
